(19) Europäisches Patentamt
European Patent Office
Office européen des brevets

(11) Publication number: **0 430 108 A2**

# (12) EUROPEAN PATENT APPLICATION

(21) Application number: **90122437.8**

(51) Int. Cl.$^5$: **A61K 31/70**

(22) Date of filing: **24.11.90**

The title of the invention has been amended (Guidelines for Examination in the EPO, A-III, 7.3).

(30) Priority: **27.11.89 JP 304644/89**

(43) Date of publication of application: **05.06.91 Bulletin 91/23**

(84) Designated Contracting States: **DE FR GB IT Bulletin**

(71) Applicant: **NIPPON KAYAKU KABUSHIKI KAISHA**
**11-2, Fujimi 1-chome Chiyoda-ku**
**Tokyo 102(JP)**

(72) Inventor: **Shimada, Nobuyoshi**
**2-9-10, Shimo, Kita-ku**
**Tokyo(JP)**
Inventor: **Takahashi, Katsutoshi**
**3-10-8-512, Kamiya, Kita-ku**
**Tokyo(JP)**
Inventor: **Azuma, Masanobu**
**2-3, Midorigaoka**
**Asahikawa-shi, Hokkaido(JP)**
Inventor: **Sakuma, Takashi**
**Eruesute Kohzohji 401, 2-105, Kohzohji-cho**
**Kasugai-shi, Aichi-ken(JP)**

(74) Representative: **Türk, Gille, Hrabal**
**Brucknerstrasse 20**
**W-4000 Düsseldorf 13(DE)**

## (54) Use of oxetanocin-G for the treatment of varicella-zoster virus infections.

(57) This invention relates to use of oxetanocin-G or a pharmacologically acceptable salt thereof for treating varicella-zoster virus infection.

EP 0 430 108 A2

# USE OF OXETANOCIN-G FOR ANTI-VARICELLA-ZOSTER VIRUS

Background of the Invention:

Varicella-zoster virus causes zoster in infants and aged persons which is usually accompanied by neuralgia or pain.

Antiviral agents such as aciclovip are now used for treating the aforesaid disease. However some strains resistant against these antiviral agents have appeared and thus the use of these chemicals could not always achieve a satisfactory effect.

Problems to be Solved by the Invention:

Therefore it has been urgently required to develop a chemical which shows an antiviral effect on varicella-zoster virus, in particular, a drug-resistant strain.

The oxetanocin-G to be used in the present invention is a known compound described in, for example, Japanese Patent Laid-Open No. 100192/1989 (EP-$A_2$-291917) which discloses that this compound exerts antiviral effects on, for example, herpes simplex virus (HSV), cytomegalo virus (CMV) and hepatitis B virus (HBV). However this patent is utterly silent on the effect of oxetanocin-G on varicella-zoster virus (hereinafter referred to simply as VZV).

Summary of the Invention:

Under these circumstances, the present inventors conducted extensive studies and thus found out that oxetanocin-G (hereinafter referred to simply as OXT-G) represented by the following formula (1):

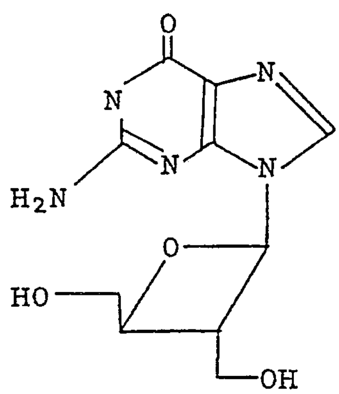

(1)

or a pharmacologically acceptable salt thereof has an anti-VZV effect and exhibits an excellent effect on a drug-resistant strain, which makes it available as an anti-VZV agent.

The present invention has been completed based on the above finding.

Accordingly, the present invention relates to a novel use of OXT-G for treating an infection of VZV or a method of treating an infection of VZV in a warm blooded animal, including human, which comprises administering an effective amount of OXT-G or a pharmacologically acceptable salt thereof to a warm-blooded animal infected with VZV to thereby suppress the growth of the VZV.

Detailed Description of the Invention:

OXT-G forms a salt together with an acid. Any pharmacologically acceptable acid may be used as the acid for forming a salt. Preferable examples of the acid include hydrochloric acid, sulfuric acid and phosphoric acid.

When OXT-G is to be used as an anti-VZV agent, it may be administered to a warm-blooded animal infected with VZV in the form of an injection, an oral preparation or a suppository either alone or as a mixture thereof with pharmaceutical additives such as filler(s) or carrier(s). The fillers and carriers may be selected from among pharmacologically acceptable ones depending on the administration route and administration method. For example, a liquid carrier such as water, an alcohol, animal or vegetable oil such

as soybean oil, peanut oil, sesame oil or a mineral oil or a synthetic oil may be used. As a solid carrier, saccharides such as maltose or sucrose, amino acids, cellulose derivatives such as hydroxypropylcellulose and organic acid salts such as magnesium stearate may be used. In the case of an injection, it is generally preferred to use physiological saline solution, various buffer solutions, solutions of saccharides such as glucose, inositol or mannitol or glycols such as ethylene glycol or polyethylene glycol. Furthermore, it is also possible to formulate OXT-G into a lyophilized preparation together with fillers such as saccharides (for example, inositol, mannitol, glucose, mannose, maltose or sucrose) or amino acids (for example, phenylalanine). The obtained lyophilized preparation is dissolved in an appropriate injection solvent such as sterilized water, physiological saline solution, glucose solution, electrolyte solution or amino acid solution suitable for intravenous administration prior to the administration.

The content of OXT-G in the preparation usually ranges from 0.1 to 100 % by weight, preferably from 1 to 90 % by weight, though it may vary depending on the type of preparation. In the case of an injection, for example, the content of OXT-G may usually range from 0.1 to 5 % by weight. In the case of oral administration, OXT-G may be formulated into, for example, tablet, capsule, dust, granules, solution or dry syrup together with the above-mentioned solid or liquid carrier(s). The capsule, tablet, granules or dust may usually contain approximately 3 to 100 % by weight, preferably 5 to 90 % by weight, of the compound of the present invention and the balance of carrier(s).

The dose may generally range from 1 to 100 mg/kg per day (in the case of parenteral administration) or from 5 to 100 mg/kg per day (in the case of oral administration), though it may be appropriately determined depending on the age, body weight and conditions of the patient as well as the purpose of the treatment.

OXT-G and a pharmacologically acceptable salt thereof are each characterized by a low toxicity and a low cumulative toxicity upon repeated administration. When 400 mg/kg of OXT-G was intraperitoneally administered to a mouse once, no symptom of intoxication could be observed.

Now Formulation Examples of the present invention will be given.

Formulation Example 1
Injection:

To 30 parts by weight of OXT-G was added purified water so as to give the total amount of 2000 parts by weight. After dissolving, the obtained solution was subjected to sterile filtration through a GS-type Millipore filter. 2 g of the filtrate thus obtained was collected in a 10-ml vial and lyophilized. Thus a lyophilized injection containing 30 mg of OXT-G per vial was obtained.

Formulation Example 2
Granules:

50 parts by weight of OXT-G, 600 parts by weight of lactose, 330 parts by weight of crystalline cellulose and 20 parts by weight of hydroxypropylcellulose were thoroughly mixed together and compressed with a roll-type compressor (Roller Compactor$^R$). Then the product was ground and the particles were dressed through 16-mesh and 60-mesh sieves to thereby give granules. Formulation Example 3
Tablet:

30 parts by weight of OXT-G, 120 parts by weight of crystalline lactose, 147 parts by weight of crystalline cellulose and 3 parts by weight of magnesium stearate were tabletted with a V-type mixer to thereby give tablets each weighing 300 mg.

Next, the anti-VZV effect of OXT-G will be described in detail by reference to the following Test Example.

Anti-VZV activity:

(1) Determination method

A monolayer of human fetal pulmonary cells, which had been grown in Eagle minimum essential medium containing 10 % of newborn calf serum, 60 µg/ml of kanamycin and 0.075 % of $NaHCO_3$, (used as a growth medium) in a 96-well microplate, was inoculated with approximately 20 PFU of various viruses and incubated at 37 °C for 2 hours. After 0.1 ml of 2 % newborn calf serum containing OXT-G was freshly supplied, the incubation was continued at 37 °C for 5 days. Plaques in each well were counted under a microscope and the ratio (%) thereof to the plaque count of a control (no addition) was calculated to prepare a graph.

Based on the graph, the median effective dose ($ED_{50}$) was determined.

The employed virus stains were as follows:

YS strain: $TK^+$ strain (carrying thymidine kinase) isolated from a patient of varicella ($TK^+$ -VZV).
YSR strain: $TK^-$ strain (carrying no thymidine kinase) obtained from the YS strain (drug-resistant strain) ($TK^-$ -VZV).

(2) Results

| Compound | Strain and $ID_{50}$* (μg/ml) | |
|---|---|---|
| | YS ($TK^+$ -VZV) | YSR ($TK^-$ -VZV) |
| OXT-G | 1.0 μg/ml | 2.0 μg/ml |

*: median inhibition dose.

Effects:

OXT-G has excellent antiviral effects on both of nonresistant and resistant strains of VZV. Thus it is expected as an excellent anti-VZV agent.

## Claims

Use of oxetanocin-G for manufacture of medicament for treating infection of varicella-zoster virus.